(19) Europäisches Patentamt
European Patent Office
Office européen des brevets

(11) Numéro de publication: **0 378 463 A1**

(12) **DEMANDE DE BREVET EUROPEEN**

(21) Numéro de dépôt: **90400047.8**

(51) Int. Cl.[5]: **C07C 211/52, C07C 209/62**

(22) Date de dépôt: **08.01.90**

(30) Priorité: **12.01.89 FR 8900309**

(43) Date de publication de la demande: **18.07.90 Bulletin 90/29**

(84) Etats contractants désignés: **AT BE CH DE ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Desmurs, Jean**
**La Jonquière, Route de Ternay**
**F-69360 Communay(FR)**
Inventeur: **Lecouve, Jean-Pierre**
**23E rue de l'Oratoire**
**F-69300 Caluire(FR)**

(74) Mandataire: **Ricalens, François et al**
**RHONE-POULENC CHIMIE Service Brevets**
**Chimie 25, quai Paul Doumer**
**F-92408 Coubevoie(FR)**

(54) **Procédé de désallylation des N-allylanilines et des N,N-diallylanilines par des métaux.**

(57) La présente invention concerne un procédé de désallylation de N-allylanilines.

Cette désallylation est effectuée en présence de métaux choisis parmi le cuivre ou le palladium dans un solvant aqueux ou alcoolique.

EP 0 378 463 A1

# PROCEDE DE DESALLYLATION DES N-ALLYLANILINES ET DES N,N-DIALLYLANILINES PAR DES METAUX

La présente invention concerne un procédé de désallylation des N-allylanilines. Elle concerne plus particulièrement un procédé de désallylation de la N,N-diallylmétatrifluorométhylaniline.

La plupart des procédés d'allylation des anilines conduisent à la formation majoritaire de monoallylaniline et à la formation secondaire souvent non négligeable de dérivés diallyliques. Lorsque l'on cherche à produire un dérivé comportant sur la fonction amine un substituant allylique et un substituant différent il est nécessaire dans une première étape de former un dérivé exclusivement monoallylé, le dérivé diallylé étant perdu. Afin de minimiser ces pertes l'industrie, dans son ensemble a cherché à limiter le taux de transformation de l'aniline en utilisant des rapports molaires dérivés allyliques/aniline fortement déficitaire en dérivés allyliques. On obtenait ainsi des taux de transformation très limités sur l'aniline qui était ensuite recyclée.

Ainsi dans notre propre demande de brevet EP 205 391 nous utilisons de préférence un rapport molaire de la métatrifluorométhylaniline à l'halogénure d'allyle d'environ 2 et nous obtenons un taux de transformation sur la métatrifluorométhylaniline de 44 %, une sélectivité de 70 % ce qui correspond à un rendement de 31 %.

Ainsi s'offre un choix limité à l'industrie, avoir un bon taux de transformation de la métatrifluorométhylaniline et obtenir une quantité non négligeable de dérivés diallyliques qu'il faut éliminer ou avoir un taux de transformation réduit de la métatrifluorométhylaniline en en mettant en oeuvre un excédant et recycler l'excès de celle-ci. La deuxième solution économiquement était la seule envisageable car la métatrifluorométhylaniline est un produit onéreux qu'aucun industriel ne veut gaspiller, mais son recyclage absolument nécessaire a toujours joué de façon négative sur le prix d'obtention de la N-allylmétatrifluorométhylaniline.

Une autre solution qui pourrait s'offrir est le recyclage de la N,N-diallylaniline après élimination d'un ou des deux groupes allyles.

En ce qui concerne les procédés de désallylation des dérivés N-allyliques et N-Ndiallylliques seulement deux procédés sont connus.

Le premier décrit par B. LAGUZZA et Coll dans Tetrahedron Letters 1981, 22 (16), 1483 consiste à déallyler des amines aliphatiques à l'aide de complexe du rhodium. Ce document est le seul qui décrive la désallylation de composés diallyliques comme le reconnaît D. PICQ, M. COTTIN, D. ANKER et H. PACHECO dans Tetrahedron Letters 1983, 24, 1399. Le procédé est néanmoins limité à la désallylation de dérivés aliphatiques qui présentent une basicité supérieure aux anilines. Le rhodium est en outre un catalyseur fort onéreux que l'industrie aimerait éviter.

Le deuxième procédé de désallylation des composés allyliques est décrit par MORI et BAN dans Chem. Pharm. Bull, 1976, 24 (9), 1981. Il consiste à mettre en présence des amides allylés avec une quantité stoechiométrique d'un catalyseur à base de palladium et de cuivre. L'utilisation de quantités stoechiométriques de palladium rend ce procédé aussi peu envisageable d'un point de vue économique que le fait de rejetter la N,N-diallylmétatrifluorométhylaniline.

La présente invention a permis de résoudre l'ensemble des problèmes laissés par l'art antérieur c'est-à-dire qu'elle a pu aboutir à un procédé économiquement viable évitant l'emploi de catalyseur au rhodium et permettant de récupérer à partir des N,N-diallylanilines l'aniline de départ et le dérivé monoallylé.

Elle a pour objet un procédé de désallylation caractérisé en ce qu'on met en contact une N-allylaniline et/ou une N,N-diallylaniline avec une quantité catalytique d'un métal choisi parmi le palladium et le cuivre dans un solvant alcoolique ou, dans l'eau ou dans un solvant hydroalcoolique.

Ce procédé est particulièrement adapté à la désallylation des allylanilines de formule générale (I) suivante :

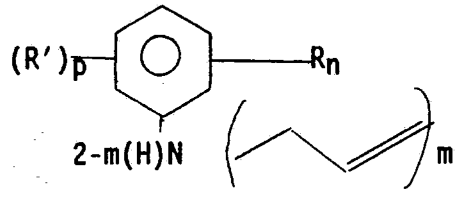

dans laquelle :

- R représente un groupe choisi de préférence parmi les radicaux attracteurs d'électrons tels que les

groupes halogénés, perhalogénoalkyle, perhalogénoalcoxy, perhalogénothioalkyle, nitro, cyano, carboxyle (-COOH), carboxyle estérifié, sulfoxyde, sulfone, sulfonique etc...

- R′ représente l'hydrogène ou un groupe alkyle, aryle ou halogène,
- n et/ou p représentent un nombre entier compris entre 1 et 2,
- m est égal à 1 ou 2.

Le groupe alkyle qui représente R′ peut avoir de 1 à 4 atomes de carbone.

On peut citer parmi les composés de formule (I) les N-allylamino ou N,N-diallylamino -chlorobenzènes, -bromobenzènes, -fluorobenzènes, les N,N-diallylamino -trifluorométhoxy, -trichlorométhoxy, -tribromométhoxybenzènes, les N,N-diallylamino -trifluorométhyl, -trichlorométhyl, -tribromométhylbenzènes, les N,N diallylamino -trifluorométhylthio, -trichlorométhylthio, -tribromométhylthiobenzènes ainsi que tous les analogues chimiques dans lesquels la chaîne alkyl, alcoxy ou thioalkyl comporte plus de un atome de carbone.

On préfère dans le cadre de la présente invention utiliser comme produit de départ de formule (I) la N,N diallylmétatrifluorométhylaniline.

Bien qu'il puisse être utilisé à d'autres états d'oxydation, le métal choisi parmi le palladium et le cuivre est utilisé de préférence sous sa forme d'oxydation II. On peut ainsi utiliser les sels de palladium et de cuivre, notamment halogénures et carboxylates, les oxydes ; ils peuvent être aussi utilisés sous forme de complexes.

Parmi ces sels, oxydes et complexes on peut citer les chlorures de palladium et de cuivre, l'acétate de palladium, le chlorure de palladium complexé par le benzonitrile. On peut également utiliser le métal à son état d'oxydation zéro, sous forme de complexe comme le palladium dibenzylidène acétone.

Le solvant alcoolique ou hydroalcoolique est choisi parmi les alcools contenant 1 à 10 atomes de carbone.

Les proportions eau/alcool pouvant varier dans de très larges limites de 0 à 100 % ou lorsque les alcools ne sont pas complètement miscibles, dans les limites de la miscibilité, puisqu'il est possible de travailler uniquement dans l'eau ou uniquement dans l'alcool.

En ce qui concerne les proportions de réactifs à mettre en oeuvre pour une meilleure réalisation de l'invention. Il est préférable d'utiliser un rapport molaire du métal à la N-allylaniline compris entre 0,05 et 0,20. Pour le solvant une concentration de la N-allylaniline ou de la N,N-diallylaniline dans l'alcool comprise entre 100 et 500 g/litre est tout à fait conseillée.

La température de réaction varie avantageusement entre 80 °C et 120 °C. La durée de réaction peut elle aussi varier largement entre 2 heures et une centaine d'heures, elle sera adaptée par l'homme de l'art à la nature de l'aniline et à la température de réaction.

Il est évident à la lecture de la présente invention que la désallylation peut être effectuée sur une matière première constituée uniquement de dérivés N-mono ou N,N-diallylique ou sur une matière première constituée d'un mélange de dérivés N mono et N,N diallylique.

La présente invention sera plus complètement décrite à l'aide des exemples suivants qui ne doivent pas être considérés comme limitatifs de l'invention.

Dans les exemples suivants les abréviations :

"TT" signifie :

$$\text{taux de transformation} = \frac{\text{nombre de moles de produit transformé}}{\text{nombre de moles de produit introduit}}$$

"RT" signifie :

$$\text{rendement sur produit transformé} = \frac{\text{nombre de moles de produit désiré}}{\text{nombre de moles de produit transformé}}$$

"RR" signifie :

$$\text{rendement sur produit introduit} = \frac{\text{nombre de moles de produit désiré}}{\text{nombre de moles de produit introduit}}$$

EXEMPLE 1 à 8 - En présence de métaux

Dans un réacteur de 30 ml, on charge
- 0,72 g de N.N.Diallyl m-trifluorométhylaniline (3 mM),
- 2 ml de solvant
- 0,3 mM de catalyseur.

Le mélange est chauffé à $90^\circ$ pendant des temps variables. Après refroidissement, on ajoute 5 ml de soude (caustique) N.

Les produits organiques sont extraits par 3 x 5 ml d'éther isopropylique. La phase organique est filtrée sur clarcel et diluée à 25 ml pour être dosée par étalonnage interne en chromatographie phase gazeuse. Les exemples sont présentés dans le tableau de la page 6.

| EXEMPLES 1 à 8 | | | | | | |
|---|---|---|---|---|---|---|
| Essai | Catalyseur | Solvant | Temps/Température | TT N,N-Diallyl | RT N-Allyl | RT m-TFMA |
| Comparatif 1 | Sans | Butanol | 15 H à $90^\circ$ | 0 | 0 | 0 |
| Invention 1 | $Pd(OAc)_2$ | Butanol | 15 H à $90^\circ$ | 32 | 42 | 9,3 |
| Invention 2 | $Pd(OAc)_2$ | Butanol | 15 H à $90^\circ$ | 49,1 | 40,8 | 10,3 |
| Invention 3 | $PdCl_2$ | Butanol | 15 H à $90^\circ$ | 66,1 | 41 | 5,4 |
| Invention 4 | $CuCl_2$ | Butanol | 15 H à $90^\circ$ | 46,6 | 48,3 | 0 |
| Invention 5 | $CuCl_2$ | Butanol | 15 H à $90^\circ$ | 52 | 56,5 | 7,7 |
| Invention 6 | $CuCl_2$ | Butanol | 30 H à $90^\circ$ | 60,4 | 61,3 | 10,4 |
| Invention 7 | $CuCl_2$ | Butanol | 92 H à $90^\circ$ | 58,1 | 59,1 | 9,5 |
| Comparatif 2 | Sans | $H_2O$ | 15 H à $90^\circ C$ | 2 | 0,5 | 0 |
| Invention 8 | $Pd(DBA)_2$ | $H_2O$ | 15 H à $90^\circ C$ | 31,2 | 39 | 28,3 |

EXEMPLE 9 :

Dans un réaoteur de 30 ml, on charge :
0,72 g de N,N-diallyl m-trifluorométhylaniline (3 millimoles)
2 ml de butanol
0,3 millimoles de catalyseur

Le mélange est chauffé 15 heures à 120° C puis le traitement est effectué comme pour les essais 1 à 8.

| Catalyseur | Solvant | Temps/Température | TT N,N-diallyl | RT N-allyl | RT m-TFMA |
|---|---|---|---|---|---|
| CuCl | Butanol | 15 h à 120° | 50,7 | 75,2 | 5,8 |
| Cu° | Butanol | 15 h à 120° | 8 | 84,7 | |
| PdCl$_2$ØCN)$_2$ | Butanol | 15 h à 120° | 98 % | 13 | 12 |
| Pd(DBA)$_2$ | Butanol | 15 h à 120° | 49 | 2,6 | 21,6 |

**Revendications**

1 - Procédé de désallylation caractérisé en ce que on met en contact une N-allylaniline et/ou une N,N-diallylaniline avec une quantité catalytique d'un métal choisi parmi le palladium et le cuivre dans un solvant alcoolique dans l'eau ou dans un solvant hydroalcoolique.

2 - Procédé selon la revendication 1 caractérisé en ce que les allylanilines répondent à la formule générale (I)

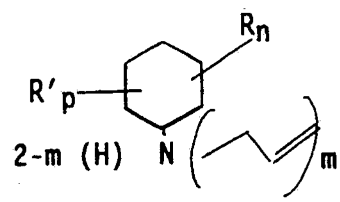

(I)

dans laquelle :
- R représente un groupe choisi parmi les radicaux halogéno, perhalogénoalkyle, perhalogénoalcoxy, perhalogénothioalkyle, nitro, cyano,
- n et/ou p représentent un nombre entier compris entre 1 et 2,
- m est égal à 1 ou 2,
- R′ représente l'hydrogène, un groupe alkyle, aryle ou halogène.

3 - Procédé selon la revendication 2 caractérisé en ce que l'allylaniline de formule (I) est la N,N-diallylmétatrifluorométhylaniline.

4 - Procédé selon la revendication 1 caractérisé en ce que le métal est sous forme de sel et de préférence choisi parmi le chlorure cuivrique, le chlorure de palladium, le diacétate de palladium, le palladium dibenzylidèneacétone.

5 - Procédé selon la revendication 1 caractérisé en ce que le solvant est choisi parmi les alcools aliphatiques contenant 1 à 10 atomes de carbone.

6 - Procédé selon la revendication 1 caractérisé en ce que le rapport molaire du métal à l'allylaniline est compris entre 0,05 et 0,20.

7 - Procédé selon la revendication 1 caractérisé en ce que la température de réaction varie entre 80° et 120° C.

8 - Catalyseur de désallylation caractérisé en ce qu'il contient au moins un élément métallique choisi parmi le palladium et le cuivre.

Office européen des brevets

# RAPPORT DE RECHERCHE EUROPEENNE

Numero de la demande

EP 90 40 0047

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | TETRAHEDRON LETTERS vol. 24, no. 13, 1983, pages 1399-1402, GB; D. PICQ et al.: "Utilisation du palladium comme catalyseur de N,N-didesallylation" * pages 1399-1402 * --- | 1,4 | C 07 C 211/52<br>C 07 C 209/62 |
| A,D | CHEMICAL AND PHARMACEUTICAL BULLETIN vol. 24, no. 9, 1976, pages 1992-1999, Tokyo, JP; M. MORI et al.: "The Reactions and Syntheses with Organometallic Compounds. II. The Deallylation Reaction with PdII and a New Synthesis of Primary Amines" * pages 1995,1997-1999 * --- | 1,4 | |
| A | CHEMICAL ABSTRACTS vol. 95, no. 15, 12 octobre 1981, page 691, colonne 1, abstract no. 133119d, Columbus, Ohio, US; J. DEHAND et al.: "Reactivity of tertiary anilines with respect to palladium complexes: dealkylation at the nitrogen and cyclopalladation"; & J. ORGANOMET. CHEM. 1981, 209 (2), pages 255-270 --- | 1,4 | **DOMAINES TECHNIQUES RECHERCHES (Int. Cl.5)** |
| A,D | TETRAHEDRON LETTERS vol. 22, no. 16, 1981, pages 1483-1486, GB; B.C. LAGUZZA et al.: "A new protecting group for amines"* page 1484 * --- | 1 | C 07 C 211/00<br>C 07 C 209/00 |
| A | US-A-4 091 019 (R.A. KEPPEL et al.) * résumé; colonne 3, lignes 14-31; revendications 1,2,4,5 * --- -/- | 1,4,5,7 | |

Le présent rapport a été établi pour toutes les revendications

| Lieu de la recherche | Date d'achèvement de la recherche | Examinateur |
|---|---|---|
| BERLIN | 07-03-1990 | RUFET J.M.A. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 90 40 0047

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | EP-A-0 205 391 (RHONE-POULENC)<br>* résumé *<br>----- | | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| BERLIN | 07-03-1990 | RUFET J.M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)